(19) Europäisches Patentamt
European Patent Office
Office européen des brevets

(11) Publication number: **0 291 007 A1**

(12) **EUROPEAN PATENT APPLICATION**

(21) Application number: 88107479.3

(22) Date of filing: 10.05.88

(51) Int. Cl.4: C07D 277/36 , A61K 31/425

(30) Priority: 11.05.87 JP 115477/87

(43) Date of publication of application: 17.11.88 Bulletin 88/46

(84) Designated Contracting States: AT BE CH DE ES FR GB GR IT LI LU NL SE

(71) Applicant: KAKEN PHARMACEUTICAL CO., LTD.
No. 28-8, 2-chome, Honkomagome
Bunkyo-Ku
Tokyo 113(JP)

(72) Inventor: Ohishi, Yoshitaka
102-10 Minamiura Ogura-cho
Uji-shi Kyoto-fu(JP)
Inventor: Mukai, Teruo
40-71, Omuro Terada
Joyo-shi Kyoto-fu(JP)
Inventor: Nagahara, Michiko
591, Oaza-Nagahara Yasu-cho
Yasu-gun Shiga-Ken(JP)
Inventor: Yajima, Motoyuki
31--406, Ohira 2-chome
Otsu-chi Shiga-ken(JP)
Inventor: Kajikawa, Norio
32-13, Takehananishinokuchi-cho
Yamashina-ku
Kyoto-shi Kyoto-fu(JP)
Inventor: Tohno, Masao
2-4, tsukinowa 3-chome
Otsu-shi Shiga-ken(JP)
Inventor: Konishi, Yoshiaki
23, Ayasugikahara Hirao Yamashiro-cho
Soraku-gun Kyoto-fu(JP)

(74) Representative: Türk, Gille, Hrabal
Bruckner Strasse 20
D-4000 Düsseldorf 13(DE)

(54) **Rhodanine derivative, process for preparing the same and pharmaceutical composition containing the same.**

(57) A rhodanine derivative having the formula (I):

EP 0 291 007 A1

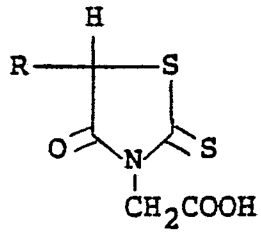

(I)

wherein R is a branched alkyl group having 5 to 7 carbon atoms or a cycloalkylalkyl group having 5 to 7 carbon atoms which may be substituted by a halogen atom; or a nontoxic salt thereof. The rhodanine derivatives (I) and their nontoxic salts have excellent aldose reductase inhibiting activity and low toxicity, and therefore can be used as therapeutic agent for diabetic complications.

# RHODANINE DERIVATIVE, PROCESS FOR PREPARING THE SAME AND PHARMACEUTICAL COMPOSITION CONTAINING THE SAME

The present invention relates to rhodanine derivatives or nontoxic salts thereof, processes for preparing the same and a pharmaceutical composition containing the same as an active ingredient which is suitable for use as a therapeutic agent for diabetic complications.

As therapeutic agents for diabetes, insulin and blood sugar lowering agents have so far been used widely. However, diabetes is not a mere disorder of sugar metabolism but a disease also involving a variety of complications and therefore the therapeutic effects of the above-mentioned agents alone are not enough for the treatment of diabetes. Pharmaceutical compositions for treatment of diabetic complications are disclosed in, for instance, Japanese Unexamined Patent Publication No. 28074/1982, No. 40478/1982, No. 136575/1985 and No. 53271/1986, and the like.

The diabetic complications include a diabetic neuropathy, a diabetic nephropathy and vascular disturbances. Diseases of the eye such as retinopathy and cataract are also important diabetic complications and form the primary cause of blindness of the aged. In the development of such diseases, not only microangiopathy is an important pathogenic factor, but also a certain kind of sugar metabolism disorder is concerned [K. H. Gabbay, Advance in Metabolic disorders (Adv. Metab. Disord.), 2(2), 424(1973)]. Thus, in the diabetic condition, polyols such as sorbitol and galactitol are accumulated to an extraordinary extent, causing osmotic pressure increase and water retention, which lead to tissue disturbance. Therefore, inhibition of aldose reductase which is essential to polyol synthesis can possibly be effective in the prevention and treatment of the above-mentioned diseases [R. G. Judzemitsch et al., New England Journal of Medicine (New Eng. J. Med.), 308, 119 to 125(1983); J. H. Kinoshita et al., Metabolism, 28(1), 462 to 469(1979)].

It is an object of the present invention to provide a rhodanine derivative having not only an excellent aldose reductase inhibiting activity but also having low toxicity.

An another object of the present invention is to provide a process for easily preparing the rhodanine derivatives or their nontoxic salts in high yields.

A further object of the present invention is to provide a pharmaceutical composition as a therapeutic agent for diabetic complications including perceptual disorder, autonomic disturbance, diabetic nephropathy, and ocular diseases such as retinopathy and cataract.

The above and other objects of the present invention will become apparent from the description hereinafter.

In accordance with the present invention, there is provided a rhodanine derivative having the formula (I):

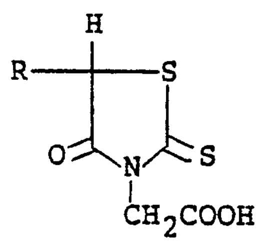

(I)

wherein R is a branched alkyl group having 5 to 7 carbon atoms or a cycloalkylalkyl group having 5 to 7 carbon atoms which may be substituted by a halogen atom; or a nontoxic salt thereof.

Also, according to the present invention, there is provided a process for preparing a rhodanine derivative having the formula (I) or a nontoxic salt thereof which comprises reducing a compound having the formula (II):

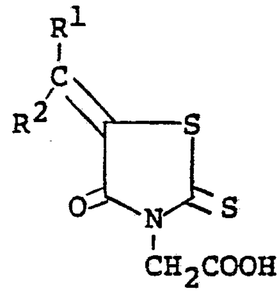

(II)

wherein $R^1$ is a hydrogen atom or an alkyl group having 1 to 3 carbon atoms and $R^2$ is an alkyl group having 2 to 6 carbon atoms, provided that when $R^1$ is a hydrogen atom, $R^2$ is a branched alkyl group having 4 to 6 carbon atoms or a cycloalkyl or cycloalkylalkyl group having 4 to 6 carbon atoms, and each $R^1$ and $R^2$ may be substituted by a halogen atom, or its salt.

Further, according to the present invention, there is provided a pharmaceutical composition as a therapeutic agent for diabetic complications which comprises as an active ingredient a rhodanine derivative having the formula (I) or a nontoxic salt thereof.

The rhodanine derivatives (I) of the present invention are excellent in activity for inhibiting accumulation of sorbitol in tissues of nerves, internal organs and so on, activity for decreasing accumulated galactitol in red blood cells, improving activity of the nerve conduction velocity, inhibiting activity of the progress of cataract, and the like, and have low toxicity. Also, the pharmaceutical composition of the invention is a therapeutic agent containing the rhodanine derivative (I) as an active ingredient, which is effective in main diabetic complications including perceptual disorder, autonomic disturbance, diabetic nephropathy and ocular diseases such as retinopathy and cataract.

The present invention provides a rhodanine derivative having the formula (I):

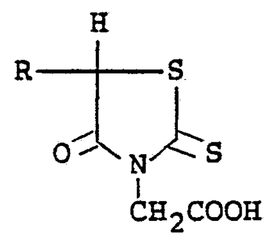

(I)

wherein R is a branched alkyl or a cycloalkylalkyl group having 5 to 7 carbon atoms which may be substituted by a halogen atom, and a nontoxic salt thereof.

In case that the number of carbon atoms of the group R is outside the above-mentioned range, the yields are lowered on the preparation of the rhodanine derivatives (I) and the medical activities of the rhodanine derivatives (I) are lowered. In case that the number of carbon atoms is from 5 to 7, there is no disadvantages as mentioned above.

Examples of the group R in the formula (I) are, for instance, a branched alkyl group such as 1-ethylpropyl group, 1,2-dimethylpropyl group, 1-ethylbutyl group, 1-propylbutyl group, 2,2-dimethylpropyl group, 3-methylbutyl group, 2-methylbutyl group or 2-ethylbutyl group; a branched alkyl group substituted by a halogen atom such as 1-ethyl-3-chloropropyl group or 1-methyl-4-chlorobutyl group; a cycloalkylalkyl group such as cyclohexylmethyl group, and the like.

Examples of the nontoxic salts of the rhodanine derivative (I) are pharmaceutically acceptable salts, for instance, metal salts such as sodium salt, potassium salt, magnesium salt and calcium salt; salts with amine compounds such as methylamine and morpholine; and the like.

Examples of the rhodanine derivatives having the formula (I) of the present invention are shown in Table 1.

Table 1

| Compound No. | Formula | Name |
|---|---|---|
| 1 | 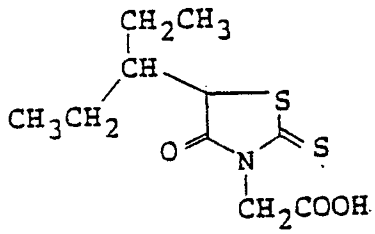 | 3-Carboxymethyl-5-(1-ethylpropyl)rhodanine |
| 2 | 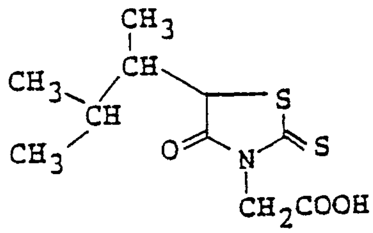 | 3-Carboxymethyl-5-(1,2-dimethylpropyl)-rhodanine |
| 3 | 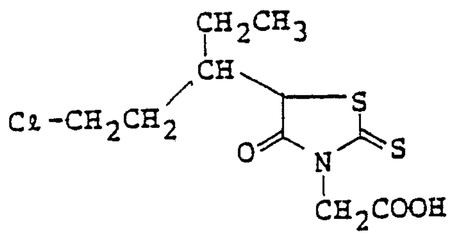 | 3-Carboxymethyl-5-(1-ethyl-3-chloropropyl)-rhodanine |
| 4 | 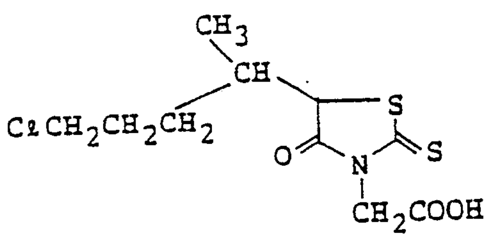 | 3-Carboxymethyl-5-(1-methyl-4-chlorobutyl)-rhodanine |
| 5 | 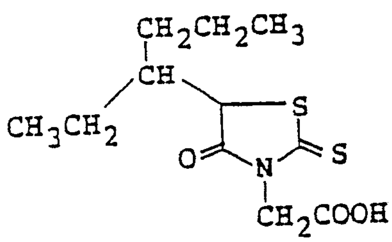 | 3-Carboxymethyl-5-(1-ethylbutyl)rhodanine |

- continued -

- continued -

| Compound No. | Formula | Name |
| --- | --- | --- |
| 6 | 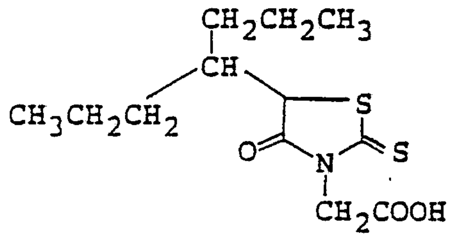 | 3-Carboxymethyl-5-(1-propylbutyl)rhodanine |
| 7 | 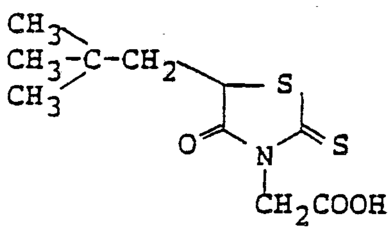 | 3-Carboxymethyl-5-(2,2-dimethylpropyl)rhodanine |
| 8 | 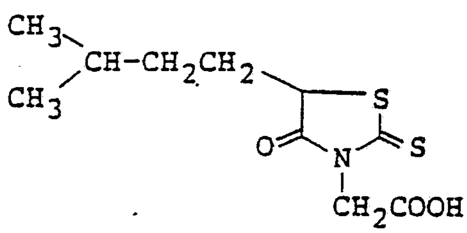 | 3-Carboxymethyl-5-(3-methylbutyl)rhodanine |
| 9 | 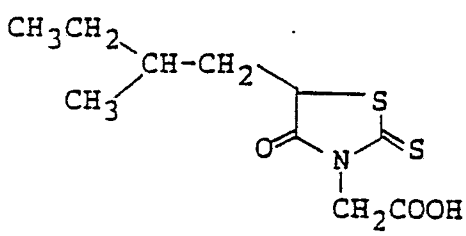 | 3-Carboxymethyl-5-(2-methylbutyl)rhodanine |
| 10 | 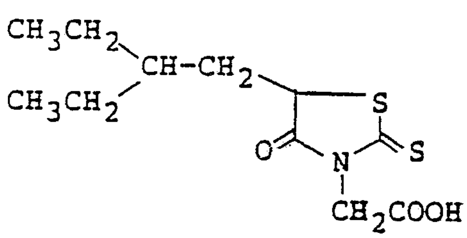 | 3-Carboxymethyl-5-(2-ethylbutyl)rhodanine |

- contiuned -

- continued -

| Compound No. | Formula | Name |
|---|---|---|
| 11 | 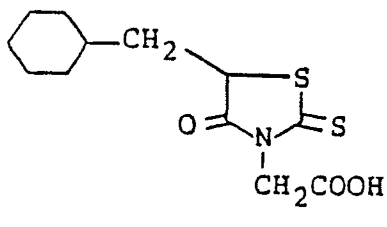 | 3-Carboxymethyl-5-cyclohexylmethylrhodanine |

The rhodanine derivatives (I) or their salts are prepared by reducing the compounds (II) or their salts. The compound used in the present invention having the formula (II):

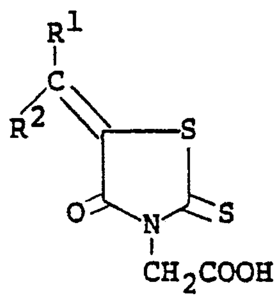

(II)

wherein $R^1$ is a hydrogen atom or an alkyl group having 1 to 3 carbon atoms and $R^2$ is an alkyl group having 2 to 6 carbon atoms, provided that when $R^1$ is a hydrogen atom, $R^2$ is a branched alkyl group having 4 to 6 carbon atoms or a cycloalkyl or cycloalkylalkyl group having 4 to 6 carbon atoms, and each $R^1$ and $R^2$ may be substituted by a halogen atoms, or a salt thereof can be prepared for instance, by reacting 3-carboxymethylrhodanine having the formula (III):

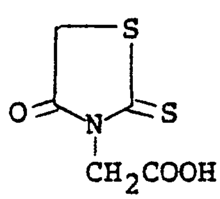

(III)

or its salt with a carbonyl compound having the formula (IV):

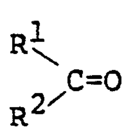

(IV)

wherein $R^1$ is a hydrogen atom or an alkyl group having 1 to 3 carbon atoms, $R^2$ is an alkyl group having 2 to 6 carbon atoms, provided that when $R^1$ is a hydrogen atom, $R^2$ is a branched alkyl group having 4 to 6 carbon atoms or a cycloalkyl or cycloalkylalkyl group having 4 to 6 carbon atoms, and each $R^1$ and $R^2$ may

be substituted by a halogen atom in the presence of a basic catalyst in a solvent.

That is, 3-carboxymethylrhodanine (III) or its salt is dissolved in a solvent such as dimethylformamide (hereinafter referred to as "DMF"), acetic acid, ethanol, dimethyl sulfoxide (hereinafter referred to as "DMSO"), ethyl acetate or acetone. The solvent may be used alone or as an admixture thereof. The basic catalyst such as sodium acetate or potassium carbonate is added to the solution of the 3-carboxymethyl-rhodanine (III) or its salt in an amount of 0.5 to 5 moles per mole of the 3-carboxymethylrhodanine (III) or its salt. To the mixture is added dropwise a carbonyl compound (IV) such as an aldehyde or a ketone at a temperature of 20° to 75° C, preferably from 25° to 35° C, in an amount of 1 to 6 moles per mole of the 3-carboxymethylrhodanine (III) or its salt. The obtained mixture is reacted at a temperature of 20° to 150° C, preferably from 40° to 100° C. After the reaction is completed, the catalyst and the solvent are removed from the reaction mixture, and then recrystallization, column chromatography and the like is conducted to give the desired compound (II). The obtained compound (II) includes any of a racemic modification and a mixture of diastereomers.

The reduction of the compound (II) is conducted preferably in the following manners: The compound (II) is dissolved in a polar protic solvent including a lower alcohol such as methanol, ethanol or propanol, or a polar aprotic solvent such as DMF or DMSO. A metallic reducing agent such as $NaBH_4$, $NaBH_3CN$, $LiAlH_4$ or $NaAlH(OC_2H_5)_3$ is added to the solution of compound (II) in an amount of 1 to 3 moles per mole of the compound (II) at a temperature of -20° to 30° C. The mixture is stirred at a temperature of -20° to 30° C, preferably from -5° to 10° C for 30 minutes for 5 hours. Then, the reaction mixture is poured into an acidic medium such as a HCl aqueous solution while cooling to form a precipitate. The precipitate is separated by filtration and recrystallized from a solvent such as ethanol, methanol, dichloromethane, dichloroethane, a mixture of ethyl acetate and water or a mixture of ethanol and water, to give the rhodanine derivative (I).

The compound (I) can be also prepared by subjecting the compound (II) to catalytic hydrogenation. For instance, the compound (II) is dissolved in a solvent such as methanol, ethanol, acetic anhydride or acetic acid, or a mixture thereof, and a catalyst such as palladium/carbon is added to the solution of compound (II) in a weight ratio of the catalyst to the compound (II) of 0.01 to 0.1. The mixture is stirred vigorously in hydrogen atomospher at 20° to 100° C, preferably from 25° to 70° C, for 30 minutes to 10 hours under a pressure from atmospheric pressure to 150 atmospheres. The catalyst is removed from the reaction mixture and the solvent is distilled off to give a crude product. The crude product is recrystallized from a solvent such as ethanol, methanol, dichloromethane, dichloroethane, ethyl acetate, water or a mixture of ethanol and water to give the rhodanine derivative (I).

The rhodanine derivatives (I) and nontoxic salts thereof of the present invention have potent aldose reductase inhibiting activity and very low toxicity. Therefore, they can serve as excellent therapeutic agents for diabetic complications.

The rhodanine derivatives (I) and nontoxic salts thereof can be formulated into pharmaceutical compositions in the form of tablets, capsules, injections, powders, pills, granules, suppositories and eye drops. Such preparations can be conducted in a usual manner using conventional pharmaceutically acceptable carries. Examples of the carrier are, for instance, binders, diluents and lubricants. Typical examples thereof are lactose, starch, sugar, microcrystalline cellulose, magnesium stearate, silicon dioxide, talc, physiological salt solution and sterilized water.

The pharmaceutical compositions are administered orally or administered as injections, suppositories, eye drops, or the like.

The effect on such prevention and treatment can be sufficiently exhibited by the dosage of about 60 to 600 mg/day, preferably from 100 to 300 mg/day, to adult.

The concentration of the rhodanine derivative (I) contained in the pharmaceutical composition such as the therapeutic agent for diabetic complications as the active ingredient can be varied within the range of 30 to 80 % by weight.

Japanese Unexamined Patent Publications No. 28074/1982 and No. 40478/1982 disclose some rhodanine derivatives for use as aldose reductase inhibitors. However, there is a substantial difference between the rhodanine derivatives disclosed in the above-cited publications and the rhodanine derivatives of the present invention in the substituent at position 5. Also, the rhodanine derivatives of the invention having the formula (I) wherein R is the branched alkyl group or cycloalkylalkyl group is more excellent in activity in vivo than rhodanine derivatives having the formula (I) wherein R is a linear alkyl group.

Further, Japanese Unexamined Patent Publication No. 136574/1985 also discloses rhodanine derivatives and nontoxic salts thereof for use as aldose reductase inhibitors. However, the rhodanine derivatives of the present invention are excellent in oral asborption, show drug effeciacy in in vivo screening, have low toxicity thereby causing hardly disturbances of a gastoric mucosa or a hepar, excellent aldose reductase inhibiting

activity in comparison with the rhodanine derivatives described in the above Publication. Accordingly, the rhodanine derivatives and its nontaxic salts of the present invention can be used as novel and highly safe therapeutic agents for diabetic complications.

Also, according to the process of the present invention, the useful rhodanine derivatives and nontoxic salts thereof as mentioned above can be easily produced in a high yield.

The present invention is more specifically described and explained by means of the following Examples. It is to be understood that the present invention is not limited to the Examples, and various changes and modifications may be made in the invention without departing from the spirit and scope thereof.

In Examples, the compound No. corresponds to the compound No. shown in Table 1.

Example 1

[Preparation of 3-carboxymethyl-5-(1-ethylpropyl)-rhodanine (Compound No. 1)]

After 10 g (0.0524 mole) of 3-carboxymethylrhodanine was dissolved in a mixed solvent of 10 mℓ of acetic acid and 50 mℓ of DMF, 11.6 g (0.0524 x 2.7 mole) of sodium acetate (hereinafter referred to as "AcONa") was added thereto and the mixture was heated at 60 °C for 30 minutes. To the resulting mixture 11.5 g (0.0524 x 2.7 mole) of diethyl ketone was added dropwise at 65 °C, which was stirred at 90 °C for 5 hours. After completion of the reaction, the solvent was distilled away from the reaction mixture, an aqueous solution of HCℓ was added to the residue, and extraction was carried out with ether. The crude product obtained from the ether layer was recrystallized from a mixture of cyclohexane and ethyl acetate (5:1 by volume) to give 6.7 g of 3-carboxymethyl-5-(1-ethylpropylidene)rhodanine (yield: 49.3 %).

In 30 mℓ of DMF was dissolved 6.7 g (0.0259 mole) of the obtained compound, to which 0.98 g (0.0259 mole) of $NaBH_4$ was added at -1 °C, and the reaction was carried out at 0° to 5 °C for 2 hours. The precipitation, which was obtained by pouring the resultant into icewater of hydrochloric acid, was recrystallized from dichloroethane to give 6.1 g of 3-carboxymethyl-5-(1-ethylpropyl)rhodanine (yield: 91 %).

Also, 20 mℓ of 0.1 N NaOH was added to 0.5 g of 3-carboxymethyl-5-(1-ethylpropyl)rhodanine to dissolve, and immediately it was freeze-dried to give its sodium salt (melting point (mp): 227° to 231 °C).

Example 2

[Preparation of 3-carboxymethyl-5-(1,2-dimethyl-propyl)rhodanine (Compound No. 2)]

There was prepared 3-carboxymethyl-5-(1,2-dimethylpropylidene)rhodanine from 3-carboxymethylrhodanine and methyl isopropyl ketone in the same manner as in Example 1.

Then, the procedure of Example 1 was repeated except that 6.7 g (0.0259 mole) of the obtained compound was dissolved in 30 mℓ of DMF, to which 0.98 g (0.0259 mole) of $NaBH_4$ was added at 0 °C to give 5.6 g of 3-carboxymethyl-5-(l,2-dimethylpropyl)rhodanine (yield: 84 %).

Also, its sodium salt (mp: 210° to 212 °C) was obtained in the same manner as in Example 1.

Example 3

[Preparation of 3-carboxymethyl-5-(1-ethyl-3-chloropropyl)rhodanine (Compound No. 3)]

There was prepared 3-carboxymethyl-5-(1-ethyl-3-chloropropylidene)rhodanine from 3-carboxymethyl-rhodanine and ethyl chloroethyl ketone in the same manner as in Example 1.

Then, the procedure of Example 1 was repeated except that 8.8 g (0.03 mole) of the obtained compound was dissolved in 50 mℓ of DMF, to which 1.14 g (0.03 mole) of $NaBH_4$ was added at -5° C to give 5.9 g of 3-carboxymethyl-5-(1-ethyl-3-chloropropyl)rhodanine (yield: 67 %).

Also, its sodium salt (mp: 230° to 236° C) was obtained in the same manner as in Example 1.

Example 4

[Preparation of 3-carboxymethyl-5-(1-methyl-4-chlorobutyl)rhodanine (Compound No. 4)]

There was prepared 3-carboxymethyl-5-(1-methyl-4-chlorobutylidene)rhodanine from 3-carboxymethyl-rhodanine and methyl chloropropyl ketone in the same manner as in Example 1.

Then, the procedure of Example 1 was repeated except that 8.8 g (0.03 mole) of the obtained compound was dissolved in 50 mℓ of DMF, to which 1.14 g (0.03 mole) of $NaBH_4$ was added at -5° C to give 5.1 g of 3-carboxymethyl-5-(1-methyl-4-chlorobutyl)rhodanine (yield: 58 %).

Also, its sodium salt (mp: 265° to 266° C) was obtained in the same manner as in Example 1.

Example 5

[Preparation of 3-carboxymethyl-5-(1-ethylbutyl)rhodanine (Compound No. 5)]

There was prepared 3-carboxymethyl-5-(1-ethylbutylidene)rhodanine from 3-carboxymethylrhodanine and ethyl propyl ketone in the same manner as in Example 1.

Then, the procedure of Example 1 was repeated except that 6.2 g (0.02 mole) of the obtained compound was dissolved in 50 mℓ of DMF, to which 0.76 g (0.02 mole) of $NaBH_4$ was added at 0° C to give 5.9 g of 3-carboxymethyl-5-(1-ethylbutyl)rhodanine (yield: 95 %).

Also, its sodium salt (mp: 270° to 275° C) was obtained in the same manner as in Example 1.

Example 6

[Preparation of 3-carboxymethyl-5-(1-propylbutyl)-rhodanine (Compound No. 6)]

There was prepared 3-carboxymethyl-5-(1-propylbutylidene)rhodanine from 3-carboxymethylrhodanine and di-n-propyl ketone in the same manner as in Example 1.

Then, 13.1 g (0.039 mole) of the obtained compound was dissolved in 50 mℓ of DMF, to which 1.7 g (0.0456 mole) of $NaBH_4$ was added at -2° to 0° C. The mixture was reacted at 0° to 2° C for 1 hour, and the resulting mixture was poured into ice-water to form a precipitate. The obtained precipitate was recrystallized from dichloroethane to give 12.3 g of 3-carboxymethyl-5-(1-propylbutyl)rhodanine (yield: 93.9 %).

Also, its sodium salt (mp: 280° or higher) was obtained in the same manner as in Example 1.

Example 7

[Preparation of 3-carboxymethyl-5-(2,2-dimethylpropyl)-rhodanine (Compound No. 7)]

To a solution of 10 g (0.0524 mole) of 3-carboxymethylrhodanine in 10 mℓ of DMF were added 8.5 mℓ of acetic acid and 11.6 g (0.0524 x 2.7 mole) of AcONa, to which 6.8 g (0.0524 x 1.5 mole) of tert-butyl aldehyde was added at 25° C. After the mixture was stirred at 80° to 115° C for 5 hours, the precipitate was filtered off from the reaction mixture. The solvent was distilled away from the obtain filtrate, diluted hydrochloric acid was poured into the residue to give a precipitate. The obtained precipitate was recrystallized from a mixture of ethanol and water, or dichloroethane to give 11.3 g (0.0437 mole) of 3-carboxymethyl-5-2,2-dimethyl propylidene)rhodanine (yields: 83 %).

The obtained compound was dissolved in 50 mℓ of DMF in an amount of 11.3 g (0.0437 mole), to which 1.7 g (0.0436 mole) of $NaBH_4$ was added at -3° to 0° C, and the mixture was stirred at -3° to 0° C for 1 hour. The reaction mixture was poured into ice-water and acidified with diluted hydrochloric acid to give a precipitate. The obtained precipitate was recrystallized from a mixture of ethanol and water to give 10.7 g of 3-carboxymethyl-5-(2,2-dimethylpropyl)rhodanine (yield: 94.7 %).

Also, its sodium salt (mp: 258° to 263° C) was obtained in the same manner as in Example 1.

Example 8

[Preparation of 3-carboxymethyl-5-(3-methylbutyl)-rhodanine (Compound No. 8)]

There was prepared 3-carboxymethyl-5-(3-methylbutylidene)rhodanine from 3-carboxymethylrhodanine and 3-methylbutyl aldehyde in the same manner as in Example 7.

The obtained rhodanine was dissolved in 50 mℓ of DMF in an amount of 11.3 g (0.0436 mole), to which 1.7 g (0.0436 mole) of $NaBH_4$ was added at -2° to 0° C. After stirring the mixture at -2° to 0° C for 1 hour, the resulting mixture was poured into ice-water and acidified with diluted hydrochloric acid to give a precipitate. The obtained precipitate was recrystallized from dichloroethane to give 10.1 g of 3-carboxymethyl-5-(3-methylbutyl)rhodanine (yield: 89.4 %).

Also, its sodium salt (mp: 280° C or higher) was obtained in the same manner as in Example 1.

Example 9

[Preparation of 3-carboxymethyl-5-(2-methylbutyl)-rhodanine (Compound No. 9)]

There was prepared 3-carboxymethyl-5-(2-methylbutylidene)rhodanine from 3-carboxymethylrhodanine and 2-methylbutyl aldehyde in the same manner as in Example 7.

The obtained rhodanine was dissolved in 50 mℓ of DMF in an amount of 11.3 g (0.0436 mole), to which 1.7 g (0.0436 mole) of $NaBH_4$ was added at -2° to 0° C. The mixture was stirred at -2° to 0° C for 1 hour, then the resulting mixture was poured into ice-water and acidified with diluted hydrochloric acid to give a precipitate. The obtained precipitate was recrystallized from a mixture of ethanol and water to give 10.9 g of 3-carboxymethyl-5-(2-methylbutyl)rhodanine (yield: 96.5 %).

Also, its sodium salt (mp: 280° C or higher) was obtained in the same manner as in Example 1.

Example 10

[Preparation of 3-carboxymethyl-5-(2-ethylbutyl)rhodanine (Compound No. 10)]

There was prepared 3-carboxymethyl-5-(2-ethylbutylidene)rhodanine from 3-carboxymethylrhodanine and 2-ethylbutyl aldehyde in the same manner as in Example 7.

The obtained rhodanine was dissolved in 50 mℓ of DMF in an amount of 11.3 g (0.0411 mole), to which

1.6 g (0.0411 mole) of $NaBH_4$ was added at -2° to 0° C. After stirring the mixture at -2° to 0° C for 1 hour, the resulting mixture was poured into iced water and acidified with diluted hydrochloric acid to give a precipitate. The obtained precipitate was recrystallized from a mixture of ethanol and water to give 9.5 g of 3-carboxymethyl-5-(2-ethylbutyl)rhodanine (yield: 84.1 %).

Also, its sodium salt (mp: 280° C or higher) was obtained in the same manner as in Example 1.

Example 11

[Preparation of 3-carboxymethyl-5-cyclohexylmethyl-rhodanine (Compound No. 11)]

There was prepared 3-carboxymethyl-5-cyclohexylmethylidenerhodanine from 3-carboxymethyl-rhodanine and cyclohexanecarboxyaldehyde in the same manner as in Example 7.

The obtained rhodanine was dissolved in 50 mℓ of DMF in an amount of 11.3 g (0.0396 mole), to which 1.5 g (0.0396 mole) of $NaBH_4$ was added at -2° to 0° C. After stirring the mixture at -2° to 0° C for 1 hour, the resulting mixture was poured into iced water and acidified with diluted hydrochloric acid to give a precipitate. The precipitate was recrystallized from dichloroethane to give 8.3 g of 3-carboxymethyl-5-cyclohexylmethylrhodanine (yield: 73.5 %).

Also, its sodium salt (mp: 253° to 257° C) was obtained in the same manner as in Example 1.

Melting point (mp), proton nuclear magnetic resonance ($^1H$-NMR) and Infrared spectrum (IR) of each of compound 1 to 11 are shown in Table 2.

Table 2

| Compound No. | formula | mp (°C) | $^1$H-NMR (δ, ppm) | IR ($CM^{-1}$) |
|---|---|---|---|---|
| 1 | 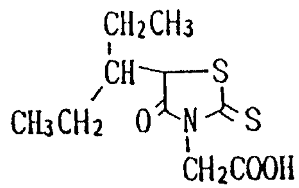 | 105 - 107 | 0.90(3H,t,$CH_3$), 1.00(3H,t,$CH_3$), 1.10 to 1.80(4H,m,$CH_2$x2), 1.95 to 2.40(1H,m,CH), 4.50(1H,d,5-H), 4.75(2H,s,$NCH_2$), 10.39(1H,brs,OH), ($CDCl_3$) | 2913(OH), 1708 (C=0) |
| 2 | 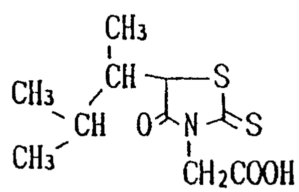 | 143 - 147 | 0.87(3H,d,$CH_3$), 0.98(6H,d,$CH_3$x2), 1.25 to 2.50(2H,m,CHx2), 4.51(1H,d,5-H), 4.77(2H,s,$NCH_2$), 10.34(1H,brs,OH), ($CDCl_3$) | 2879(OH), 1707 (C=0) |
| 3 | 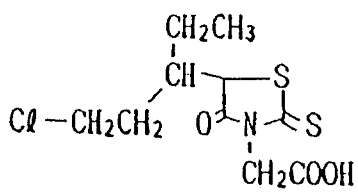 | 121 - 123 | 0.91(3H,t,$CH_3$), 1.10 to 1.76(6H,m, $CH_2$x3), 2.05 to 2.38(1H,m,CH), 4.60(1H,d,5-H), 4.79(2H,s,$NCH_2$), 8.25(1H,brs,OH), ($CDCl_3$) | 2990(OH), 1701 (C=0) |

- continued -

- continued -

| Compound No. | formula | mp (°C) | $^1$H-NMR (δ, ppm) | IR ($CM^{-1}$) |
|---|---|---|---|---|
| 4 | 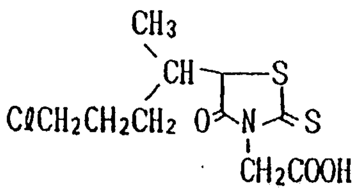 | 153 - 157 | 0.88(3H,d,$CH_3$), 1.12 to 1.90(6H,m,$CH_2$x3), 1.90to 2.40(1H,m,CH), 4.52(1H,d,5-H), 4.81(2H,s,$NCH_2$), 10.10(1H,brs,OH), ($CDCl_3$) | 2885(OH), 1729(C=0) |
| 5 | 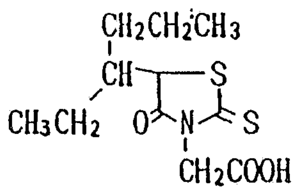 | 95 - 101 | 0.93(6H,t,$CH_3$x2), 1.10 to 1.70(6H,m,$CH_2$x3), 2.15 to 2.45(1H,m,CH), 4.50(1H,d,5-H), 4.78(2H,s,$NCH_2$), 10.16(1H,brs,OH), ($CDCl_3$) | 2891(OH), 721(C=0) |
| 6 | 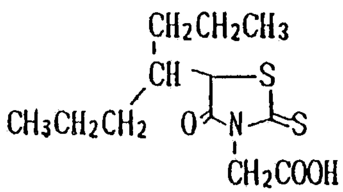 | 119 - 121 | 0.92(6H,t,$CH_3$x2), 1.10 to 1.80(8H,m,$CH_2CH_2$x2), 2.10 to 2.60(1H,m,CH), 4.49(1H,d,5-H), 4.80(2H,s,$NCH_2$), 10.58(1H,s,OH), ($CDCl_3$) | 2920(OH), 1709(C=0) |

- continued -

- continued -

| Compound No. | formula | mp (°C) | $^1$H-NMR (δ, ppm) | IR ($CM^{-1}$) |
|---|---|---|---|---|
| 7 | 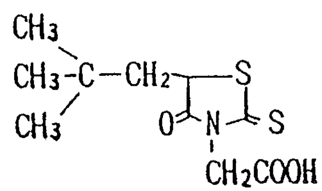 | 166 - 167 | 1.02(9H,s,$CH_3$x3), 1.77(1H,dd,$CCH_2$x$\frac{1}{2}$), 2.36(1H,dd,$CCH_2$x$\frac{1}{2}$), 4.48(1H, dd,5-H), 4.70(2H,s,$NCH_2$), 7.30(1H, brs,OH), (acetone-$d_6$) | 2850(OH), 1713(C=O) |
| 8 | 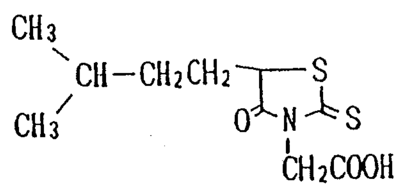 | 105 - 118 | 0.93(6H,d,$CH_3$x2), 1.10 to 2.55(5H,m, $CH_2CH_2CH$), 4.29(1H,dd,5-H), 4.80(2H, s,$NCH_2$), 10.11(1H,s,OH), ($CDCl_3$) | 2900(OH), 1710(C=O) |
| 9 | 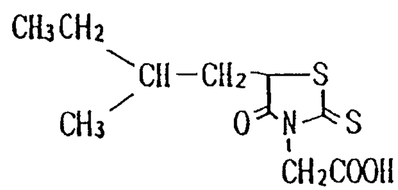 | 118 - 120 | 0.90(3H,t,$C\underline{H}_3CH_2$), 0.94(3H,d,$C\underline{H}_3CH$), 1.10 to 1.80(3H,m,$CH_3C\underline{H}_2$ and CH), 1.80 to 2.25(2H,m,>$CHC\underline{H}_2$) 4.50(1H,d,5-H), 4.68(2H,s, $NCH_2$), 8.85(1H,brs,OH), (acetone-$d_6$) | 2850(OH), 1721(C=O) |

- continued -

- continued -

| Compound No. | formula | mp (°C) | $^1H$-NMR (δ, ppm) | IR ($CM^{-1}$) |
|---|---|---|---|---|
| 10 | 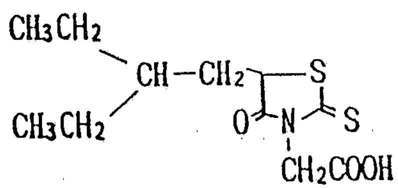 | 124 - 126 | 0.88(6H,t,$CH_3$x2), 1.10 to 1.65(5H,m, $CH_3C\underline{H}_2$x2 and CH), 1.70 to 2.30(2H,m, $>CHC\underline{H}_2$), 4.33(1H,dd,5-H), 4.72(2H,s, $NCH_2$), 9.73(1H, brs,OH), ($CDCl_3$) | 2900(OH), 1728(C=0) |
| 11 | 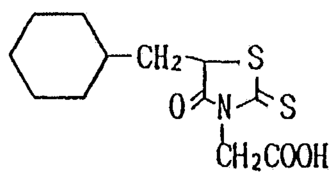 | 149 - 151 | 0.90 to 1.80(11H,m,H in cyclohexane ring), 1.80 to 2.15(2H,m,$C_6H_{11}C\underline{H}_2$), 4.52(1H,d,5-H), 4.71(2H,s,$NCH_2$), 8.55(1H,brs,OH), (acetone-$d_6$) | 2895(OH), 1713(C=0) |

The rhodanine derivatives (I) and their nontoxic salts of the present invention have various medical activities as mentioned below.

Example 12

[Aldose reductase inhibiting activity]

The rhodanine derivatives (I) and their nontoxic salt of the present invention have the aldose reductase inhibiting activity and are useful for prevention and treatment of diabetic neuropathy, diabetic nephropathy, diabetic retinopathy or cataract, which is caused by accumulating polyols such as sorbitol to an extraordinary extent.

Examples of the nontoxic salt of the rhodanine derivative (I) of the present invention are, for instance, alkali metal salts such as sodium salt and potassium salt, alkaline earth metal salts such as calcium salt and barium salt, various salts with organic amines, and the like.

The aldose reductase inhibiting activity of the rhodanine derivatives (I) of the invention was examined according to the method of Hayman et al [S. Hayman and J. H. Kinoshita, J. Biol. Chem., 240, 877 to 882 (1965)].

As the result, in the laboratory test for inhibiting activity against aldose reductase obtained from a rat lens, all Compound Nos. 1 to 11 obtained in Examples 1 to 11 showed 50 % inhibition within the concentration range of $10^{-8}$ to $10^{-7}$M.

Example 13

[Inhibiting activity of the accumulation of sorbitol in nervous tissues]

The rhodanine derivatives of the present invention inhibit aldose reductase activity resulting in decrease of the amount of sorbitol accumulated in nervous tissues involving hyperglycemia. Consequently, they can improve the depression of cell functions, whereby diabetic complications such as diabetic neuropathy can be cured. Actually, the rhodanine derivatives and the salt thereof of the present invention not only can inhibit aldose reductase activity effectively in vitro, but also can decrease the amount of sorbitol accumulated in the tissues of test animals suffering from diabetes which are experimentally produced.

The inhibiting activity of the accumulation of sorbitol in nervous tissues was examined with respect to the rhodanine derivatives (I) of the present invention.

Test Method

1) Process for producing rats suffering from diabetes used in the test

After preliminary breeding SD male rats (6 weeks old) in this laboratory for 5 days, streptozotocin (made by Sigma Chemical Company) was administered intravenously (45 mg/kg body weight) to the rats to destroy β cells of Langerhans' islets in pancreas. Thus, rats suffering from diabetes were obtained.

2) Administration of the rhodanine derivative and excision of nervous tissue of the rats

After 48 hours from the administration of streptozotocin, the rhodanine derivative suspended in a 5 % aqueous solution of gum arabic was administered orally (100 mg/kg body weight or 25 mg/kg body weight) to the rats suffering from diabetes.

On the other hand, a 5 % aqueous solution of gum arabic having no rhodanine derivative was administered orally (5 mℓ/kg body weight) to the rats (a control group).

After 5 hours from the administration, the rats were sacrificed by the decapitation and bled to death. Immidiately, the sciatic nervous tissues of the rats were excised and frozen with dry ice. The excised sciatic nervous tissues were stored at -70 °C until the sorbitol amount was measured.

3) Extraction and measurement of sorbitol

After the wet weights of nervous tissues were measured, the tissues were homogenized in 1.0 mℓ of distilled water by using a homogenizer, to which 0.2 mℓ of a 4.5 % aqueous solution of barium hydroxide and 0.2 mℓ of a 5 % aqueous solution of zinc sulfate were added. The mixture was centrifuged for 10 minutes at 3,000 rpm to remove a precipitate. Ion exchange resins "Dowex 50 W" (Dow Chemical Co.) and "Duolite A-4" (Sumitomo Chemical Company, Limited.) purchased were added in small amounts to the obtained supernatant to desalt, which was recentrifuged to obtain a supernatant. To 0.5 mℓ of the extract of sorbitol were added 1.0 mt of a 50 mM glycine-sodium hydroxide buffer solution (pH 9.4) containing 0.2 mM nicotinamide adenine dinucleotide (NAD) and 50 μℓ of an enzyme solution containing 83 μg of sorbitol dehydrogenase (commercially available from BOEHRINGER MANNHEIM CORPORATION), and the mixture was reacted at room temperature for one hour. The amount of NADH formed by oxidation of sorbitol was measured by using a spectrofluorimeter and a sorbitol concentration in the extracts was measured. From the obtained results, sorbitol concentrations in nervous tissues (n mole/mg wet weight) were calculated.

Results

In rats suffering from diabetes, sorbitol was accumulated in the nervous tissues thereof. That is, the rat suffering from diabetes had a sorbitol content of 0.81 ± 0.06 n mole/mg in its sciatic nervous tissues. On the other hand, the normal rat had a sorbitol content of 0.12 ± 0.01 n mole/mg, and therefore, the rat suffering from diabetes had about 7 times sorbitol content of the normal rat.

On the other hand, when the rhodanine derivative of the present invention was administered to the rats suffering from diabetes, aldose reductase activity in the nervous tissue thereof was inhibited to produce sorbitol, thus resulting in remarkable decreasing of the amount of sorbitol accumulated in the tissue.

A difference between the sorbitol content of the normal rat (0.12 ± 0.01 n mole/mg) and that of the rat suffering from diabetes (0.81 ± 0.06 n mole/mg) was defined as an amount of sorbitol accumulated in the tissues due to diabetes (hereinafter referred to as "sorbitol amount due to diabetes"). Also, a difference between the sorbitol content of the rat suffering from diabetes (0.81 ± 0.06 n moles/mg) and the sorbitol content of the rat suffering from diabetes which was administered the rhodanine derivative was defined as a lowering of the amount of sorbitol due to administration of the rhodanine derivative (hereinafter referred to as "lowering of sorbitol").

A lowering percentage (%) was defined as a percentage of the lowering of sorbitol to the sorbitol amount due to diabetes. The lowering percentages of the rhodanine derivatives (Compound Nos. 1-11) are shown in Table 3 (administered 100 mg/kg body weight) and Table 4 (administered 25 mg/kg body weight) with the number of rats suffering from diabetes.

Table 3

| Compound No. | Lowering percentage (%) | The number of rats suffering from diabetes (rats) |
|---|---|---|
| 1 | 51.5 ± 4.8*[3] | 6 |
| 2 | 68.2 ± 10.6*[3] | 5 |
| 3 | 62.4 ± 9.4*[3] | 5 |
| 4 | 60.1 ± 7.3*[3] | 5 |
| 5 | 30.9 ± 8.8*[1] | 5 |
| 6 | 28.4 ± 7.4*[1] | 5 |
| 7 | 80.9 ± 5.8*[3] | 5 |
| 8 | 23.0 ± 6.2*[1] | 6 |
| 9 | 49.9 ± 6.9*[2] | 5 |
| 10 | 42.9 ± 7.6*[2] | 6 |
| 11 | 27.7 ± 1.6*[1] | 5 |

(Notes) *1: $p < 0.05$, *2: $p < 0.01$, *3: $p < 0.001$
(Dunnett's t-test: differences from control)

Table 4

| Compound No. | Lowering percentage (%) | The number of rats suffering from diabetes (rats) |
|---|---|---|
| 1 | 19.8 ± 3.2* | 6 |
| 2 | 36.5 ± 4.7* | 6 |
| 3 | 34.0 ± 6.2* | 6 |
| 4 | 32.2 ± 4.4* | 6 |
| 5 | 15.2 ± 2.1* | 6 |
| 6 | 16.1 ± 3.2* | 6 |
| 7 | 52.5 ± 3.4* | 6 |
| 8 | 12.2 ± 1.8* | 6 |
| 9 | 19.0 ± 3.5* | 6 |
| 10 | 18.2 ± 2.0* | 6 |
| 11 | 13.0 ± 2.9* | 6 |

(Note) *: $p < 0.05$ (Dunnett's t-text: differences from control)

From the results of Table 3 and Table 4, it was confirmed that all Compounds Nos. 1-11 efficiently decreased the amount of accumulated sorbitol in nervous tissues.

On the other hand, with respect to rhodanine compounds having at 5-position linear alkyl groups such as 3-carboxymethyl-5-n-butylrhodanine, 3-carboxymethyl-5-n-propylrhodanine and 3-carboxymethyl-5-n-heptylrhodanine, these compounds hardly inhibited the accumulation of sorbitol. That is, it was confirmed that the compounds having the branched alkyl group or cycloalkylalkyl group at 5-position have an excellent aldose reductase inhibiting activity.

Example 14

[Improving activity of the nerve conduction velocity]

As aforementioned, the rhodanine derivatives (I) of the present invention had the inhibiting activity of the accumulation of sorbitol in nervous tissues. That is, the rhodanine derivatives (I) can recover the depression of functions of nervous tissues due to the accumulation of sorbitol. Accordingly, it is expected that the rhodanine derivatives (I) can improve the conduction velocity of nerve.

The improving activity of the nerve conduction velocity was examined with respect to rats suffering from diabetes.

Test Method

Rats suffering from diabetes for the test were prepared in the same manner as in Example 13.

From the day after the administration of streptozotocin, each of Compound Nos. 1-11 suspended in a 5 % aqueous solution of gum arabic was administrated to the rats in a dosage of 100 mg/kg body weight once daily for 2 weeks.

As the control groups, a 5 % aqueous solution of gum aratic having no rhodanine derivative was administered (5 mℓ/kg body weight) in the same manner as above to the rats.

Two weeks later, the conduction velocity of nerve of the rats was measured in accordance with the method of Miyoshi [Fukuoka Medical Jounal, 62(7), 588 to 603(1971)]. In this test, the nerves of the rats' tails were used as materials studied.

The rats were anesthetized with pentobarbital (30 to 45 mg/kg body weight, intraperitoneal administration), and then the conduction velocities of nerves were measured in a constant-temperature box having a temperature of 40 °C.

The nerve of rat's tail was pricked with needle electrodes at a portion of about 1 cm from the anus (the first stimulation point) and at a portion of about 5 cm in a distal direction from the first stimulation point (the second stimulation point). Further, the segmental muscle of the rat's tail was pricked with a coaxial needle electrode at a portion of about 5 cm in a distol direction from the second stimulation point. The longitudinal nerve trunk of the rat's tail was stimulated at the first stimulation point and the second stimulation point to record action potentials of the provoked muscle.

The conduction velocity of nerve was calculated as follows:

$$\text{Conduction velocity of nerve (m/sec)} = \frac{\text{(A distance between the first stimulation point and the second stimulation point)}}{\text{(A difference between the time occupied on the rising up of the action potential by the stimulation of the first stimulation point and that by the stimulation of the second stimulation point)}}$$

Also, with respect to the normal rats administered with a 5 % aqueous solution of gum arabic, the conduction velocity of nerve was measured in the same manner as above.

Results

The results are shown in Table 5.

Table 5

| Rat | Compound No. | Conduction velocity of nerve (m/sec) | The number of rats suffering from diabetes (rats) |
|---|---|---|---|
| Rat suffering from diabetes | 1 | 36.2 ± 3.4* | 6 |
| " | 2 | 36.6 ± 2.7* | 6 |
| " | 3 | 34.2 ± 1.8* | 6 |
| " | 4 | 37.0 ± 3.5* | 6 |
| " | 5 | 32.4 ± 2.8* | 6 |
| " | 6 | 33.0 ± 3.1* | 6 |
| " | 7 | 38.5 ± 4.0* | 6 |
| " | 8 | 35.2 ± 1.5* | 6 |
| " | 9 | 33.6 ± 2.6* | 6 |
| " | 10 | 34.1 ± 4.3* | 6 |
| " | 11 | 31.8 ± 1.3* | 6 |
| Control group | - | 24.5 ± 1.2 | 6 |
| Normal rat | - | 38.4 ± 2.2 | 6 (normal rats) |

(Note) *: $p < 0.05$ (Dunnett's t-test: differences from control)

From the results of Table 5, it would be obvious that all of Compound Nos. 1-11 could effectively improve the nerve conduction velocity of the rats suffering from diabetes.

Example 15

[Inhibiting activity of the progress of cataract]

The rhodanine derivatives (I) of the present invention can be used in the treatment for diabetic cataract due to their excellent inhibing activity of the accumulation of sorbitol.

The inhibiting activity of the progress of cataract was examined with respect to the rhodanine derivative (I).

Test Method

Rats suffering from diabetes for the test were produced in the same manner as in Example 13.

From the day after the administration of streptozotocin, each of Compound Nos. 1-11 suspended in a 5 % aqueous solution of gum arabic was administered orally 100 mg/kg body weight daily for 5 weeks to the rats. As the control group, a 5 % aqueous solution of gum arabic having no rhodanine derivative was administered (5 mℓ/kg body weight) to the rats in the same manner as above.

After administering streptozotocin, lenses of the rats were observed by using a slit lump (commercially available from KOWA Co., Ltd. under a trade name "Kowa SL-2") every second or third day for 5 weeks, sufficiently keeping the lenses mydriasis with a mydriatic (commercially available from Santen Pharmaceutical Co., Ltd. under the trade name "MYDRIN-P".

The opaqueness of the lenses was estimated according to the following criteria.

Score 0: There was no change in the lenses.

Score 1: Opacity was observed in the state of a vacuole around the equator lentis.

Score 2: Opacity was observed at the center of lens' surface but the fundus oculi was seen as if projected through the lens.

Score 3: Opacity was observed over the whole of lens' surface and the fundus oculi was not seen as if projected through the lens.

Score 4: The nucleus lentis became opaque.

Results

Each average value of the obtained the scores of both eyes in the rats is shown in Table 6 as the opaqueness.

Also, with respect to the normal rats administered with a 5 % aqueous solution of gum arabic, the opaqueness were estimated in the same manner as above. The result is also shown in Table 6.

Table 6

| Rats | Compound No. | Opaqueness | The Number of rats suffering from diabetes (rats) |
|---|---|---|---|
| Rats suffering from diabetes | 1 | 0.98 ± 0.06* | 6 |
| " | 2 | 0.61 ± 0.07* | 6 |
| " | 3 | 0.55 ± 0.06* | 6 |
| " | 4 | 0.68 ± 0.09* | 6 |
| " | 5 | 0.92 ± 0.10* | 6 |
| " | 6 | 0.90 ± 0.12* | 6 |
| " | 7 | 0.32 ± 0.05* | 6 |
| " | 8 | 0.89 ± 0.10* | 6 |
| " | 9 | 0.72 ± 0.11* | 6 |
| " | 10 | 0.74 ± 0.12* | 6 |
| " | 11 | 0.90 ± 0.13* | 6 |
| Control group | - | 1.46 ± 0.12 | 6 |
| Normal rats | - | 0 | 6 (normal rats) |

(Note) *: $p < 0.05$ (Dunnett's t-test: differences from control)

The results of Table 6 suggest the possibility of a clinical application of the rhodanine derivatives (I) of the present invention in the treatment of diabetic cataract.

Example 16

[Inhibiting activity of the accumulation of galactitol in red blood cells

Galactose is reduced by the aldose reductase to form galactitol. The rhodanine derivatives (I) of the present invention inhibit the aldose reductase activity resulting in decrease of the amount of galactitol accumulated in even living bodies having a large amount of galactose.

The inhibiting activity of the accumulation of galactitol was examined with respect to the rhodanine derivatives (I) of the invention.

Test Method

Each of Compound Nos. 1-11 was administered orally in a dosage of 10 mg/kg body weight once daily for 3 days with a capsule (administration time: 9 a.m. to 9:30 a.m.) to a male or female beagle dogs (body weight: from 8 to 12 kg). As the control group, the dogs were administered with vacant capsules in the same manner as above. Dog foods containing galactose in an amount of 15 g/kg body weight were given to

the dogs immediately after the final administration of the capsule. The dogs completely had eaten the dog foods for 5 to 10 minutes. The blood was collected from the cephalic vein of the dog using heparine sodium after 10 hours from the administration of galactose. The collected blood was centrifuged to give red blood cells. The obtained red blood cells were washed twice with a cold physiological saline solution and were hemolysed in distilled water having an equal volume to the red blood cells.

After removing protein from the obtained solution, the resulting solution was treated with ion exchange resins, "Dowex 50 W" and "Duolite A-4", desalted and lyophilized. After subjecting to the pre-treatment of galactitol with trimethyl silyl ether (TMS), the amount of galactitol accumulated in the red blood cells was measured by a gas chromatography.

Result

The results are shown in Table 7.

Table 7

| Compound No. | Amount of galactitol in red blood cells (ng/g hemoglobin) | The number of dogs (dogs) |
|---|---|---|
| 1 | 82 ± 6* | 3 |
| 2 | 66 ± 5* | 3 |
| 3 | 57 ± 12* | 3 |
| 4 | 59 ± 6* | 3 |
| 5 | 94 ± 7* | 3 |
| 6 | 86 ± 12* | 3 |
| 7 | 48 ± 3* | 3 |
| 8 | 72 ± 11* | 3 |
| 9 | 69 ± 10* | 3 |
| 10 | 90 ± 10* | 3 |
| 11 | 87 ± 13* | 3 |
| Control group | 125 ± 10 | 3 |

(Note) *: $p < 0.05$ (Dunnett's t-test: differences from control)

From the results of Table 7, it would be understood that the rhodanine derivatives (I) of the invention can effectively inhibit not only the accumulation of sorbitol but also the accumulation of galactitol.

Example 17

[Acute toxicity]

The acute toxicity in mice was examined by using the rhodanine derivatives of compound Nos. 1, 2, 5, 7 and 10.

Test Method

To groups of 4 male ddY mice 5 weeks old were orally administered by force each test compound suspended in a 10 % aqueous solution of gum arabic, in amounts of 500 mg, 1,000 mg and 2,000 mg per kg body weight, and the mice were observed for 2 weeks to count the number of dead mice in each amount.

Results

With respect to Compound Nos. 1, 2, 5, 7 and 10, no dead mice were observed up to a dose of 2,000 mg/kg body weight, and therefore the $LD_{50}$ (oral) of each compound was more than 2,000 mg/kg body weight. Accordingly, it was demonstrated that the rhodanine derivative (I) showed a very low toxicity.

As the above results indicate, the rhodanine derivatives (I) of the present invention have an excellent activities such as aldose reductase inhibiting activity, improving activity of the nerve conduction velocity and inhibiting activity of the progress of cataract in both *in vivo* and *in vitro*, and can be used as novel and highly safe therapeutic agents for diabetic complications.

Example 18

[Preparation of therapeutic agents]

The rhodanine derivatives (I) and the nontoxic salt thereof according to the present invention can be used as therapeutic agents for diabetic complications by orally administered. The prescriptions of the agents for oral administration are as follows:

(1) According to the following prescription, tablets of 200 mg/tablet were prepared.

| Components | mg |
|---|---|
| 3-Carboxymethyl-5-(2,2-dimethylpropyl)rhodanine | 200 |
| Lactose | 25 |
| Cornstarch | 45 |
| Crystalline cellulose | 15 |
| Methylcellulose | 3 |
| Calcium stearate | 2 |

(2) According to the following prescription, components were mixed, and capsules were prepared by filling each capsule No. 5. with 200 mg of the mixed components.

| Components | mg |
|---|---|
| 3-Carboxymethyl-5-(2,2-dimethylpropyl)rhodanine | 110 |
| Lactose | 45 |
| Cornstarch | 35 |
| Crystalline cellulose | 8 |
| Calcium stearate | 2 |

The prescriptions are not limited thereto.

In addition to the ingredients used in the Examples, other ingredients can be used in the Examples as set forth in the specification to obtain substantially the same results.

**Claims**

1. A rhodanine derivative having the formula (I):

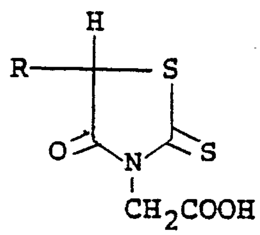

(I)

wherein R is a branched alkyl group having 5 to 7 carbon atoms or a cycloalkylalkyl group having 5 to 7 carbon atoms which may be substituted by a halogen atom; or a nontoxic salt thereof.

2. The rhodanine derivative of Claim 1, which is a member selected from the group consisting of 3-carboxymethyl-5-(1-ethylpropyl)rhodanine, 3-carboxymethyl-5-(1,2-dimethylpropyl)rhodanine, 3-carboxymethyl-5-(1-ethyl-3-chloropropyl)rhodanine, 3-carboxymethyl-5-(1-methyl-4-chlorobutyl)rhodanine, 3-carboxymethyl-5-(1-ethylbutyl)rhodanine, 3-carboxymethyl-5-(1-propylbutyl)-rhodanine, 3-carboxymethyl-5-(2,2-dimethylpropyl)-rhodanine, 3-carboxymethyl-5-(3-methylbutyl)rhodanine, 3-carboxymethyl-5-(2-methylbutyl)rhodanine, 3-carboxymethyl-5-(2-ethylbutyl)rhodanine and 3-carboxymethyl-5-cyclohexylmethyl-rhodanine; and nontoxic salts thereof.

3. A process for preparing a rhodanine derivative having the formula (I):

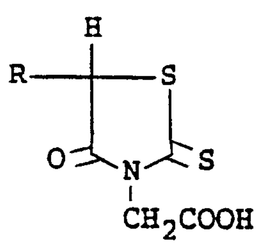

(I)

wherein R is a branched alkyl group having 5 to 7 carbon atoms or a cycloalkylalkyl group having 5 to 7 carbon atoms which may be substituted by a halogen atom; or a nontoxic salt thereof, which comprises reducing a compound having the formula (II):

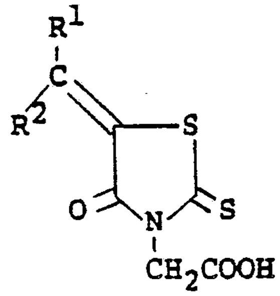

(II)

wherein $R^1$ is a hydrogen atom or an alkyl group having 1 to 3 carbon atoms and $R^2$ is an alkyl group having 2 to 6 carbon atoms, provided that when $R^1$ is a hydrogen atom, $R^2$ is a branched alkyl group having 4 to 6 carbon atoms or a cycloalkyl or cycloalkylalkyl group having 4 to 6 carbon atoms, and each $R^1$ and $R^2$ may be substituted by a halogen atom or its salt.

4. The process of Claim 3, wherein said compound having the formula (II) is a member selected from the group consisting of 3-carboxymethyl-5-(1-ethylpropylidene)rhodanine, 3-carboxymethyl-5-(1,2-dimethylpropylidene)rhodanine, 3-carboxymethyl-5-(1-ethyl-3-chloropropylidene)rhodanine, 3-carboxymethyl-5-(1-methyl-4-chlorobutylidene)rhodanine, 3-carboxymethyl-5-(1-ethylbutylidene)rhodanine, 3-carboxymethyl-5-(1-propylbutylidene)rhodanine, 3-carboxymethyl-5-(2,2-dimethylpropylidene)rhodanine, 3-carboxymethyl-5-(3-methylbutylidene)rhodanine, 3-carboxymethyl-5-(2-methylbutylidene)rhodanine, 3-carboxymethyl-5-(2-ethylbutylidene)rhodanine and 3-carboxymethyl-5-cyclohexylmethylidenerhodanine; and nontoxic salts thereof.

5. A pharmaceutical composition as a therapeutic agent for diabetic complications which comprises as an active ingredient a rhodanine derivative having the formula (I):

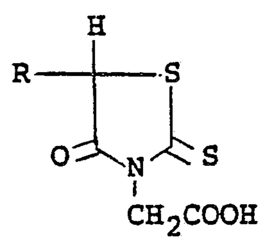

(I)

wherein R is a branched alkyl group having 5 to 7 carbon atoms or a cycloalkylalkyl group having 5 to 7 carbon atoms which may be substituted by a halogen atom; or a nontoxic salt thereof.

European Patent Office

# EUROPEAN SEARCH REPORT

Application number

EP 88107479.3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP - A1 - 0 045 165 (UNO PHARMACEUTICAL CO., LTD.)<br>* Claims 1,20-25 *<br>-- | 1,3,5 | C 07 D 277/36<br>A 61 K 31/425 |
| A | EP - A2/A3 - 0 143 461 (KAKEN PHARMACEUTICAL CO., LTD.)<br>* Claims 1,8 *<br>---- | 1,3,5 | |

| TECHNICAL FIELDS SEARCHED (Int. Cl.4) |
|---|
| C 07 D 277/00 |

The present search report has been drawn up for all claims

| Place of search | Date of completion of the search | Examiner |
|---|---|---|
| VIENNA | 26-07-1988 | BRUS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document